# FASCICULE DE BREVET EUROPEEN

(11) **EP 2 419 439 B1**
(45) Date de publication et mention de la délivrance du brevet: **02.09.2015**
(21) Numéro de dépôt: 10718239.6
(22) Date de dépôt: 15.04.2010
(51) Int. Cl.: C07K 5/103, C07K 7/06, C07K 14/395, C07K 14/415, A61K 38/01, A61K 8/64, A61P 17/00, C07K 4/10, A61K 8/97, A61Q 19/00, A61Q 17/00, A61Q 17/04, A61Q 7/00

(54) **COMPOSITION COSMÉTIQUE ET/OU PHARMACEUTIQUE COMPRENANT UN HYDROLYSAT PEPTIDIQUE APAISANT**
EIN LINDERNDES PEPTIDISCHES HYDROLYSAT ENTHALTENDE KOSMETISCHE UND/ODER PHARMAZEUTISCHE ZUSAMMENSETZUNG
COSMETIC AND/OR PHARMACEUTICAL COMPOSITION COMPRISING A RELIEVING PEPTIDIC HYDROLYSATE

(30) Priorité: 15.04.2009 FR 0901824
(43) Date de publication de la demande: 22.02.2012
(73) Titulaire: ISP Investments Inc., Wilmington, DE 19805 (US)
(72) Inventeur: DAL FARRA, Claude, Kerhonkson, New York 12446 (US); DOMLOGE, Nouha, F-06560 Valbonne (FR); BOTTO, Jean-Marie, F-06560 Valbonne (FR)
(74) Mandataire: Macquet, Christophe
(86) Numéro de dépôt international: PCT/FR2010/000312
(87) Numéro de publication internationale: WO 2010/119192

(56) Documents cités:
- EP-A- 0 265 099
- EP-A- 1 281 401
- WO-A-03/008438
- WO-A-03/068184
- WO-A-2005/107697
- FR-A- 2 925 325
- FR-A- 2 925 326
- FR-A- 2 925 327
- FR-A- 2 925 330

## Description

La présente invention se situe dans le domaine cosmétique et pharmaceutique, et plus particulièrement dans le domaine de la dermatologie. L'invention concerne une composition comprenant, dans un milieu physiologiquement acceptable, un hydrolysat peptidique enrichi en peptide bioactif apaisant. Le peptide bioactif est caractérisé en ce qu'il comprend de 4 à 6 acides aminés dont au moins un résidu glycine, un résidu leucine et un résidu acide glutamique. De préférence, le principe actif provient de l'hydrolyse de protéines de plantes choisies parmi l'épeautre, la pomme de terre, le maïs, l'orge et le colza. L'invention concerne également l'utilisation dudit hydrolysat peptidique, en tant que principe actif pour activer la HMG-CoA réductase, dans des compositions destinées à prévenir ou à lutter contre les irritations cutanées.

La fonction première de l'épiderme est de constituer une barrière entre l'environnement extérieur et le milieu intérieur. C'est la couche la plus externe de l'épiderme, *le stratum corneum*, qui assure cette fonction. Il est composé de kératinocytes au stade ultime de leur différenciation, les cornéocytes, scellés les uns aux autres par un épais ciment lipidique intercellulaire, à la fois souple et imperméable. Ce ciment lipidique contient du cholestérol, un lipide neutre activement synthétisé par les kératinocytes des couches intermédiaires de l'épiderme. L'enzyme membranaire qui joue un rôle clé dans cette synthèse est la 3-hydroxy-3-methylglutaryl- Coenzyme A (HMG-CoA) -réductase (E.C. 1.1.1.34), qui existe au moins sous deux isoformes dans la peau humaine (Luskey et al., J Biol Chem., 1985 260(18), p.10271-7).

A la suite d'une altération brutale de la barrière cutanée, on observe une augmentation importante et rapide de la synthèse de cholestérol, associée à une augmentation de l'expression et de l'activité de la HMG-CoA réductase (Menon G.K. et al., J. Lipid, Res., 1985, (26), P. 418-427). D'autre part, l'inhibition médicamenteuse, par l'administration topique de statines, confirme l'importance du cholestérol dans la fonction barrière épidermique et le rôle central de l'HMG-CoA réductase (Proksch E. et al., British J. Dermatol., 1993, (128), p. 473-482).

De nombreuses personnes souffrent de symptômes liés à une grande sensibilité de la peau. Cette sensibilité se traduit le plus souvent par des rougeurs (érythèmes), douleurs, picotements ou prurits, voir même l'apparition de boutons réactionnels. Les causes d'apparition de cette sensibilité sont multiples, mais on peut distinguer les sensibilités liées au stress, à l'absorption de certains aliments, la réactivité liées aux conditions climatiques ou encore les effets secondaires liés à l'application topique de produits irritants.

La plupart des symptômes liés à la sensibilité de la peau résulte de processus irritatifs qui mettent en jeux des réactions cellulaires localisées aboutissant à la libération de médiateurs chimiques tels que les cytokines, la substance P ou les prostaglandines. L'irritation cutanée s'accompagne généralement d'une altération de la fonction barrière.

La présente invention a pour principal objectif de fournir un nouveau principe actif apaisant, capable d'apporter une solution à la sensibilité de la peau.

Afin d'apaiser les sensations d'inconfort des peaux sensibles, il faut d'une part limiter les manifestations de l'irritation, comme les érythèmes et les picotements, en freinant la libération des médiateurs cellulaires de l'inflammation et d'autre part prévenir l'altération ou rétablir la fonction barrière de l'épiderme. Dans ce domaine particulier, l'apport direct de substituts lipidiques, tels que les céramides (EP 1272148, US2007576937) ou certains dérivés du cholestérol (FR 2 789 312), a largement été décrit. D'autre part, l'utilisation de polyphénols pour limiter la libération de médiateurs de l'inflammation a également été décrite (WO/2004/058282). Toutefois, à ce jour, aucun document ne décrit ou ne suggère l'objet de l'invention ; c'est-à-dire qu'une composition comprenant un hydrolysat peptidique enrichi en peptide' bioactif selon l'invention peut avoir des propriétés intéressantes pour apaiser les peaux sensibles et pour prévenir ou lutter contre les irritations de la peau.

Le premier objet de la présente invention est une composition cosmétique ou pharmaceutique comprenant, dans un milieu physiologiquement adapté, en tant que principe actif apaisant, une quantité efficace d'un hydrolysat peptidique enrichi en peptide bioactif capable de renforcer la fonction barrière de l'épiderme, ledit peptide bioactif contenant de 4 à 6 acides aminés dont au moins un résidu glycine, un résidu leucine et un résidu acide glutamique, en tant que principe actif activateur de la HMG-CoA réductase, pour son utilisation pour apaiser les peaux sensibles, pour protéger la peau contre les agressions extérieures, ou pour prévenir ou lutter contre les irritations cutanées. Les inventeurs ont en effet mis en évidence une activité cosmétique d'hydrolysats peptidiques contenant certains peptides particuliers, dénommés peptides bioactifs ci-après.

Il ont en particulier mis en évidence que l'hydrolysat peptidique, enrichi en peptide bioactif, lorsqu'il est appliqué sur la peau, a des propriétés apaisantes. Ces propriétés ont été démontrées par la mise en évidence que l'hydrolysat, lorsqu'il est appliqué sur la peau, protège cette dernière de divers agents irritants expérimentaux.

On entend par «peptide bioactif » selon l'invention un enchaînement d'au moins quatre acides aminés, liés entre eux par des liaisons peptidiques ou par des liaisons peptidiques modifiées et qui possède une activité *in vivo* ou *in vitro* caractéristique de l'activité du principe actif de la composition utilisée selon l'invention ».

L'activité biologique caractéristique selon l'invention est définie *in vitro* par la capacité du peptide à activer l'HMG-CoA réductase, soit par l'augmentation de la synthèse protéique de la HMG-CoA réductase (par modulation directe ou indirecte de l'expression génique de la HMG-CoA réductase), soit par l'augmentation de l'activité enzymatique de la HMG-CoA réductase, soit par d'autres processus biologiques tels que la stabilisation de la protéine HMG-CoA réductase ou encore la stabilisation des transcrits d'ARN messager.

On entend par peau, l'ensemble des tissus de recouvrement constituant la peau et les muqueuses, y compris le cuir chevelu.

On entend par « hydrolysat peptidique », un mélange de composés majoritairement représentés par des peptides ou des oligopeptides. Selon l'invention, on utilisera indifféremment les termes « hydrolysat peptidique » ou « principe actif ».

On entend par « composés de nature peptidique », les fragments de protéines, les peptides et les acides aminés libres présents dans l'hydrolysat peptidique selon l'invention.

On entend par « application topique », le fait d'appliquer ou d'étaler une composition contenant le principe actif utilisée selon l'invention à la surface de la peau ou d'une muqueuse. On entend par « physiologiquement acceptable », qu'une composition contenant l'hydrolysat peptidique utilisée selon l'invention est appropriée pour entrer en contact avec la peau ou une muqueuse sans provoquer de réactions de toxicité ou d'intolérance.

Selon une méthode particulièrement avantageuse de réalisation de l'invention, le peptide bioactif contenu dans l'hydrolysat possède une séquence de formule générale (I)

X₁-[Gly, Glu, Leu]- X₂-X₃

Dans laquelle,
X₁ est l'alanine, la valine, l'isoleucine ou aucun acide aminé,
X₂ est la serine ou la thréonine
X₃ est la leucine, l'isoleucine ou aucun acide aminé,

Selon une méthode de réalisation de l'invention tout particulièrement préférée, le peptide bioactif est de séquence :
(SEQ ID n°1) Ala-Glu-Gly-Leu-Ser-Ile
(SEQ ID n°2) Leu-Gly-Glu-Ser-Leu
(SEQ ID n°3) Val-Gly-Glu-Leu-Thr
(SEQ ID n°4) Ile-Gly-Glu-Leu-Ser
(SEQ ID n°5) Ala-Gly-Glu-Leu-Ser
(SEQ ID n°6) Gly-Glu-Leu-Thr-Ile
(SEQ ID n°7) Gly-Glu-Leu-Ser

Selon un mode de réalisation particulièrement intéressant, le peptide biologiquement actif correspond à la séquence SEQ ID n°5.

Le principe actif de la composition utilisée selon l'invention peut être obtenu par extraction de protéines d'origine végétale, suivie d'une hydrolyse contrôlée qui libère des composés de nature peptidique, parmi lesquels se trouvent les peptides bioactifs.

L'utilisation d'hydrolysats peptidiques, et en particulier d'hydrolysats peptidiques de bas poids moléculaires, présente de nombreux avantages en cosmétique. Outre le fait de générer des composés de nature peptidique qui ne préexistaient pas dans le mélange protéique de départ, l'hydrolyse et la purification permettent d'obtenir des mélanges plus stables, plus facilement standardisables et ne provocant pas de réactions allergiques en dermato-cosmétique.

Il est également possible d'utiliser certains extraits hydrolysés sans en purifier les composés de nature peptidique correspondant aux peptides bioactifs de la composition utilisée selon l'invention, mais en s'assurant toutefois de la présence desdits peptides par des moyens analytiques appropriés.

De très nombreuses protéines trouvées dans les plantes sont susceptibles de contenir des peptides bioactifs au sein de leur structure. L'hydrolyse ménagée permet de dégager ces composés de nature peptidique particuliers. Il est possible, mais non nécessaire pour réaliser l'invention, d'extraire soit les protéines concernées d'abord et de les hydrolyser ensuite, soit d'effectuer l'hydrolyse d'abord sur un extrait brut et de purifier les composés de nature peptidique ensuite.

Selon un mode de réalisation préférée, ledit principe actif provient de l'hydrolyse de protéines de plantes choisies parmi l'épeautre, la pomme de terre, le maïs, l'orge et le colza. De préférence, les plantes utilisées ne sont pas soumises à une fermentation préalable.

Ainsi, l'invention peut être réalisée en utilisant des graines d'engrain ou petit épeautre (*Triticum monococcum*), qui est un blé diploïde très ancien contenant un taux particulièrement élevé en protéines (Vallega 1992).

L'invention peut également être réalisée en utilisant des tubercules de pomme de terre du genre Solanum et plus particulièrement de l'espèce *Solanum tuberosum*. Le tubercule n'appartient pas à la racine de la plante, mais à sa tige enterrée, dont partent des rameaux plus grêles appelés rhizomes, à l'extrémité desquels se forment les tubercules.

L'invention peut également être réalisée en utilisant les graines d'une des nombreuses plantes du genre Zea et préférentiellement l'espèce *Zea mays L.* Selon l'invention le matériel végétal utilisé sera le grain et préférentiellement le grain débarrassé de son enveloppe par une étape de décorticage.

L'invention peut également être réalisée en utilisant l'une des nombreuses plantes de la famille des poacées, telle que l'orge (*Hordeum vulgare L*.). Selon l'invention le matériel végétal utilisé sera le grain et préférentiellement le grain débarrassé de son enveloppe par une étape de décorticage.

L'invention peut également être réalisée en utilisant les graines d'une des nombreuses plantes de la famille des crucifères (Cruciferae ou Brassicaceae). Comme plante utilisées selon l'invention appartenant à cette famille, on peut citer des plantes oléagineuses telles que le colza (*Brassica napus*, *var. oleifera*). Selon l'invention le matériel végétal utilisé sera le grain et préférentiellement le grain débarrassé de son enveloppe par une étape de décorticage.

Toute méthode d'extraction ou de purification connue de l'homme du métier peut être utilisée afin de préparer l'hydrolysat selon l'invention.

Dans une première étape, les graines, ou une partie spécifique de la plante (feuilles, tubercules, racines, etc.) sont broyées à l'aide d'un broyeur à plantes. La poudre ainsi obtenue peut ultérieurement être "délipidée" à l'aide d'un solvant organique classique (comme par exemple un alcool, l'hexane ou de l'acétone).

On réalise ensuite l'extraction des protéines suivant le procédé classique (Osborne, 1924) modifié ; le broyat de plante est mis en suspension dans une solution alcaline contenant un produit adsorbant de type polyvinylpolypyrrolidone (PVPP) insoluble (0,01 -20 %) ; en effet il a été observé que les opérations d'hydrolyses et de purifications ultérieures étaient facilitées par ce moyen. En particulier, la concentration en substances de type phénoliques, interagissant avec les protéines, se trouve nettement réduite.

La fraction soluble, contenant les protéines, les glucides et éventuellement des lipides, est recueillie après des étapes de centrifugation et de filtration. Cette solution brute est ensuite hydrolysée dans des conditions ménagées pour générer des peptides solubles. L'hydrolyse se définit comme étant une réaction chimique impliquant le clivage d'une molécule par de l'eau, cette réaction pouvant se faire en milieu neutre, acide ou basique. Selon l'invention, l'hydrolyse est réalisée par voie chimique et/ou de façon avantageuse par des enzymes protéolytiques. On peut alors citer l'utilisation des endoprotéases d'origine végétale (papaïne, bromelaïne, ficine) et de micro-organismes (Aspergillus, Rhizopus, Bacillus, etc.). Les conditions d'hydrolyses sont choisies pour favoriser l'enrichissement en peptide bioactif.

Pour les mêmes raisons que précédemment, c'est-à-dire l'élimination des substances polyphénoliques, une quantité de polyvinylpolypyrrolidone est additionnée au milieu réactionnel lors de cette étape d'hydrolyse ménagée. Après filtration, permettant d'éliminer les enzymes et les polymères, le filtrat (solution) obtenu constitue une première forme du principe actif selon l'invention.

L'hydrolysat obtenu à ce stade peut être encore purifié afin de sélectionner les fractions de bas poids moléculaires, préférentiellement inférieure à 6 kDa, et les peptides générés selon leur nature. Le fractionnement peut s'effectuer avantageusement par des étapes d'ultrafiltrations successives à travers des filtres de porosité décroissante, en conservant les filtrats à chaque étape et/ ou par une méthode de type chromatographique, afin d'enrichir spécifiquement l'hydrolysat en peptide bioactif.

On procède à une phase de dilution dans de l'eau ou dans tout mélange contenant de l'eau, puis stérilisée par ultrafiltration afin d'obtenir un hydrolysat peptidique caractérisé par une teneur en protéines de 0,5 à 5,5 g/l. Cet hydrolysat peptidique correspond à la forme la plus purifiée du principe actif de la composition utilisée selon l'invention.

L'hydrolysat peptidique obtenu compris dans la composition selon l'invention est analysé qualitativement et quantitativement en chromatographie liquide haute pression (CLHP), permettant d'analyser les protéines ayant des poids moléculaires de 0,2 à 25 kDa (selon un gradient de solvants approprié). Les différentes fractions peptidiques qui ont pu être isolées sont ensuite analysées pour leur efficacité biologique. Ces diverses fractions sont ensuite analysées par spectrométrie de masse afin d'identifier spécifiquement le contenu en acides aminés dès peptides de chaque pic. Il a également été réalisé une analyse de séquençage, pour déterminer la séquence peptidique du peptide bioactif.

L'hydrolysat obtenu est composé de peptides de poids moléculaire inférieur à 6 kDa préférentiellement inférieures à 6 kDa, et enrichi en peptide bioactif de 4 à 6 acides aminés comprenant au moins un résidu glycine, un résidu leucine et un résidu acide glutamique.

La quantité efficace de principe actif correspond à la quantité nécessaire pour obtenir le résultat recherché, à savoir, activer la HMG-CoA réductase, inhiber la production de médiateur cellulaire de type interleukine-1, et ainsi procurer un effet apaisant, chez les individus présentant une peau sensible. Selon certains aspects de l'invention, la quantité efficace de principe actif est une quantité suffisante de peptide de formule générale (I) pour diminuer voire supprimer un effet irritant cutané. Ainsi, cette quantité est variable en fonction de la quantité et de la nature du composé à caractère irritant utilisé et/ou de la sensibilité de l'utilisateur à ce composé.

Selon un mode de réalisation avantageux de l'invention, le principe actif de la composition utilisée selon l'invention est présent dans les compositions de l'invention en quantité efficace, soit à une concentration comprise entre 0,0001 % et 20 % environ, et préférentiellement à une concentration comprise entre 0,05 % et 5 % environ par rapport au poids total de la composition finale.

Selon un mode de réalisation avantageux de l'invention, le principe actif de la composition utilisée selon l'invention est solubilisé dans un ou plusieurs solvants physiologiquement acceptables, classiquement utilisés par l'homme du métier, comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

Selon encore un autre mode de réalisation avantageux de l'invention, le principe actif de la composition utilisée selon l'invention est préalablement solubilisé dans un vecteur cosmétique ou pharmaceutique comme les liposomes ou adsorbé sur des polymères organiques poudreux, des supports minéraux comme les talcs et bentonites, et plus généralement solubilisé dans, ou fixé sur, tout vecteur physiologiquement acceptable.

La composition utilisable selon l'invention peut en particulier consister en une composition pour soins capillaires, et notamment un shampooing, un après-shampooing, une lotion traitante, un masque, etc. Ainsi, le principe actif de la composition utilisée selon l'invention pourra avantageusement être utilisé dans les soins antipelliculaires du cuir chevelu. La composition peut également se présenter sous forme de teinture ou de mascara à appliquer au pinceau ou au peigne.

La composition utilisable selon l'invention pourra être appliquée par toute voie appropriée, notamment orale, parentérale ou topique, et la formulation des compositions sera adaptée par l'homme du métier, en particulier pour des compositions cosmétiques ou dermatologiques. Avantageusement, les compositions selon l'invention sont destinées à une administration par voie topique. Ces compositions doivent donc contenir un milieu physiologiquement acceptable, c'est-à-dire compatible avec la peau et les phanères, et couvrent toutes les formes cosmétiques ou dermatologiques.

Il est bien entendu que le principe actif de la composition utilisée selon l'invention peut être utilisé seul ou bien en association avec d'autres principes actifs.

Avantageusement, les compositions utilisables selon l'invention contiennent, en outre, divers principes actifs destinés à favoriser l'action du principe actif de la composition utilisée selon l'invention. On peut citer, de manière non limitative, les classes d'ingrédients suivantes : d'autres agents actifs peptidiques, des extraits de végétaux, des agents cicatrisants, anti-âge, antirides, apaisants, anti-radicalaires, anti-UV, des agents stimulant la synthèse de macromolécules dermiques ou le métabolisme énergétique, des agents hydratants, antibactériens, antifongiques, anti-inflammatoires, anesthésiques, des agents modulants la différenciation, la pigmentation ou la dépigmentation cutanée, des agents stimulant la pousse des ongles ou des cheveux. Préférentiellement, on utilisera un agent anti-radicalaire ou antioxydant.

Selon un autre aspect de l'invention, les compositions peuvent comprendre en outre des principes actifs à effet secondaire irritant et donc susceptibles de provoquer une irritation cutanée spécialement chez les personnes à peau sensible. Comme principes actifs susceptibles d'avoir un effet secondaire irritant, on peut citer par exemple : les agents kératolytiques tels que les a-hydroxy-acides comme les acides glycolique, lactique, malique, citrique, tartrique, mandélique, et leurs dérivés ; les ss-hydroxy-acides comme l'acide salicylique et ses dérivés ; les a-céto-acides comme l'acide ascorbique ou vitamine C et ses dérivés ; les rétinoïdes comme le rétinol et ses esters, le rétinal, l'acide rétinoïque et ses dérivés ; le minoxidil et ses dérivés ; les sels de lithium ; les teintures ou colorants capillaires comme la para-phénylène diamine (p- PDA) et certains de ses dérivés tels que la N-phenyl p-PDA et le toluène 2,5- diamine sulfate ; la méta-phénylène diamine (m-PDA) et certains de ses dérivés tels que la toluène 3,4-diamine ; l'ortho-phénylène diamine (o-PDA) ; les solutions alcooliques parfumantes (parfums, eaux de toilette, après-rasage, déodorants) ; les agents anti-transpirants (certains sels d'aluminium) ; les actifs dépilatoires ou de permanentes (thiols, ammoniaque) ; les dépigmentants (hydroquinone) ; les actifs anti-poux ; les agents détergents (ioniques et non ioniques) ; et leurs mélanges.

Ainsi, selon cet aspect de l'invention l'hydrolysat peptidique de la composition utilisée selon l'invention sera utilisé en tant que principe actif pour prévenir ou lutter contre les irritations cutanées provoquées par le principe actif irritant.

Il est bien évident que l'invention s'adresse aux mammifères en général, et plus particulièrement aux êtres humains.

Ces compositions pourront notamment se présenter sous forme d'une solution aqueuse, hydroalcoolique ou huileuse ; d'une émulsion huile-dans-eau, eau-dans-huile ou émulsions multiples ; elles peuvent aussi se présenter sous forme de crèmes, de suspensions, ou encore de poudres, adaptées à une application sur la peau, les muqueuses, les lèvres et/ou les phanères. Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la peau sous forme d'aérosol. Elles peuvent être utilisées comme produit de soin et/ou comme produit de maquillage de la peau.

L'ensemble de ces compositions comprennent, en outre, tout additif communément utilisé dans le domaine d'application envisagé ainsi que les adjuvants nécessaires à leur formulation, tels que des solvants, des épaississants, des diluants, des anti-oxydants, des colorants, des filtres solaires, des agents auto-bronzants, des pigments, des charges, des conservateurs, des parfums, des absorbeurs d'odeur, d'autres principes actifs cosmétiques, des huiles essentielles, des vitamines, des acides gras essentiels, des tensioactifs, des polymères filmogènes, etc.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre à des concentrations allant de 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Ledit principe actif, grâce à ses propriétés particulières, pourra être utilisé dans une composition cosmétique pour apaiser les peaux sensibles.

Selon un autre aspect de l'invention, l'hydrolysat peptidique pourra être utilisé avantageusement, en tant que principe actif, dans une composition cosmétique pour protéger la peau contre tous types d'agressions extérieures.

On entend par l'expression « agressions extérieures », les agressions que peut produire l'environnement. A titre d'exemple, on peut citer des agressions telles que la pollution, les UV (rayonnements ultra-violets), les oxydants, ou encore les produits à caractère irritant tels que les tensioactifs, les conservateurs, les parfums, ou certains principes actifs utilisés en dermato-cosmétologie, tels que les actifs kératolytiques, exfoliants, les alpha- hydroxy-acides (notamment acides lactique, glycolique, citrique), les 3-hydroxy-acides (notamment acides salicylique, n-octanoyl-5-salicylique) et les rétinoïdes (notamment rétinol et ses esters), les actifs anti-poux. On peut encore citer les agressions mécaniques, telles que les abrasions, le rasage ou l'épilation. Les conditions climatiques extrêmes sont également une cause importante d'agression cutanée. Ces agressions extérieures aboutissent à une altération de la fonction barrière qui se traduit par un inconfort cutané, des phénomènes sensoriels désagréables, tels que des tiraillements ou des démangeaisons, voire des fragilités excessives et des rougeurs.

En particulier, l'invention a pour objet une composition cosmétique comprenant l'hydrolysat selon l'invention pour son utilisation pour prévenir ou traiter les dommages causés à la peau par les rayonnements UV.

En particulier, l'invention a pour objet une composition comprenant l'hydrolysat selon l'invention pour son utilisation pour prévenir ou traiter les dommages causés à la peau par les oxydants.

En particulier, l'invention a pour objet une composition comprenant l'hydrolysat selon l'invention pour son utilisation pour prévenir ou traiter les dommages causés à la peau par des agressions extérieures de la peau choisies parmi les traitements mécaniques tels que le rasage ou l'épilation, les décapages trop intenses par les détergents, les conditions climatiques extrêmes ou les variations brutales de température et d'hygrométrie.

L'invention a pour objet une composition pharmaceutique comprenant une quantité efficace d'hydrolysat peptidique selon l'invention pour son utilisation pour prévenir ou traiter les inflammations cutanées, telles que les érythèmes, en particulier dus aux ultraviolets, le prurit, l'urticaire, les piqûres d'insectes, les allergies, ou encore l'alopécie dans ses phases inflammatoires.

Selon cette forme de l'invention, les compositions seront appropriées à une administration orale pour un usage pharmaceutique. Ainsi les compositions pourront notamment se présenter sous forme de comprimés, capsules, gélules, pâtes à mâcher, poudres à consommer telles quelles ou à mélanger extemporanément avec un liquide, sirop, gels, et toute autre forme connue de l'homme du métier. Ces compositions comprennent, en outre, tout additif communément utilisé dans le domaine d'application envisagé ainsi que les adjuvants nécessaires à leur formulation, tels que des solvants, des épaississants, des diluants, des antioxydants, des conservateurs, d'autres principes actifs pharmaceutiques, des huiles essentielles, des vitamines, des acides gras essentiels.

D'autres avantages et caractéristiques de l'invention apparaîtront mieux à la lecture des exemples donnés à titre illustratif et non limitatif.

### Liste des figures

Figure 1 : Exemple de chromatogramme obtenu par CLHP, avec mise en évidence du pic correspondant au peptide bioactif dans un hydrolysat d'épeautre
Figure 2 : Exemple de chromatogramme obtenu par CLHP, avec mise en évidence du pic correspondant au peptide bioactif dans un hydrolysat de pomme de terre
Figure 3 : Exemple de chromatogramme obtenu par CLHP, avec mise en évidence du pic correspondant au peptide bioactif dans un hydrolysat de maïs

### Exemple 1 : Préparation d'un hydrolysat peptidique à partir d'épeautre (Triticum monococcum)

Les grains d'épeautre (*Triticum monococcum*) sont mis en solution dans 10 volumes d'eau en présence de 2 % de POLYCLAR® 10 (polyvinylpyrrolidone - PVPP - insoluble). Le mélange est ajusté à un pH compris entre 6 et 8 avec une solution aqueuse de soude 1 M.

Après ajustement du pH, une amylase (hasidase®) et une protéase (papaïne à 2 %) sont ajoutées dans le milieu réactionnel. L'hydrolyse est obtenue après 2 heures d'agitation à 50°C. On procède ensuite à l'inactivation de l'enzyme par chauffage de la solution à 80°C pendant 2 heures. Après centrifugation, la solution aqueuse surnageante correspondant à un hydrolysat brut d'épeautre est récupérée. Les conditions d'hydrolyse ont été choisies de façon à permettre un enrichissement en peptide bioactif de 4 à 6 acides aminés contenant les résidus Gly, Leu et Acide glutamique.

Le procédé de purification de l'hydrolysat brut commence par des filtrations successives à l'aide de filtres-plaques Seitz-Orion de porosité décroissante (jusqu'à 0.2 µm) afin d'obtenir une solution brillante et limpide, qualifiée d'hydrolysat 1.

A cette étape, l'hydrolysat 1 d'épeautre est caractérisé par une couleur jaune clair et par un extrait sec titrant de 20 à 25 g/kg, un taux de protéines de 10 à 12 g/l et un taux de sucres de 5 à 8 g/l.

La nature protéique de l'hydrolysat 1 est mise en évidence après analyse par électrophorèse sur gel de polyacrylamide NuPAGE® Bis-Tris Pre-cast (Invitrogen). L'hydrolysat protéique d'épeautre est chauffé à 70°C pendant 10 minutes dans des conditions réductrices dénaturantes dans un tampon de préparation d'échantillon NuPAGE® LDS. Une solution de NuPAGE® Antioxydant est ajoutée dans la cuve intérieure (cathode) pour éviter que les protéines réduites ne se ré-oxydent durant l'électrophorèse. La migration des protéines est réalisée dans du tampon de migration NuPAGE® MES avec le standard SeeBlue Plus2 comme marqueur de poids moléculaire. La coloration des protéines est effectuée à l'aide de Bleu de Coomassie® R-250. Dans ces conditions, on observe une bande à 24 kDa, correspondant à l'enzyme, puis des protéines inférieures à 6 kDa.

L'hydrolysat 1 est ensuite purifié par ultrafiltration à l'aide de la cassette Pellicon® 2 Biomax 5 kDa afin d'éliminer toutes traces d'enzymes. En fin de purification, on obtient un hydrolysat peptidique jaune-orangé, brillant et limpide. On procède à une phase de dilution afin d'obtenir un hydrolysat peptidique caractérisé par une teneur en protéines de 1,5 à 3,5 g/l. Cet hydrolysat peptidique correspond au principe actif selon l'invention. Cet hydrolysat peptidique est alors analysé en chromatographie liquide haute pression (HPLC) à l'aide d'un appareil HP1100 piloté par le logiciel ChemStation. La colonne utilisée lors de l'élution de l'hydrolysat est une Nucleosil® 300-5 C4 MPN (125 x 4 mn), permettant de chromatographier des protéines ayant des poids moléculaires de 0,2 à 25 kDa dans les conditions suivantes :
Gradient acétonitrile
   - Colonne Uptisphere OPB 125 x 3 mm
   - Solvant A: eau grade HPLC contenant 0.1% d'acide trifluoroacétique (TFA)
   - Solvant B: acétonitrile
   - Gradient: 100 % à 1 5% solvant A en 35 min.

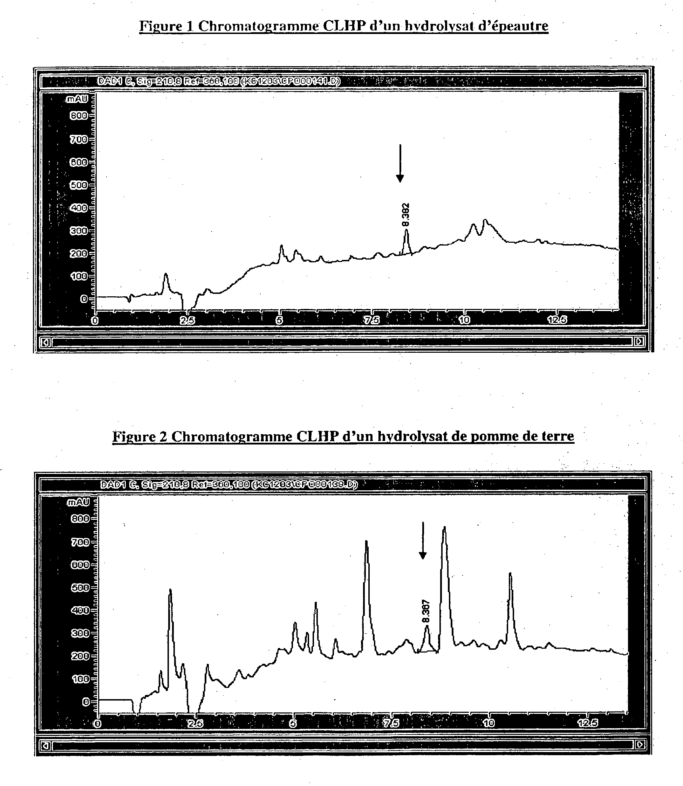

Dans ces conditions chromatographiques, plusieurs fractions peptidiques ont pu être isolées. Un exemple de chromatogramme obtenu par CLHP (chromatographie en phase liquide à haute pression), avec mise en évidence du pic correspondant au peptide bioactif est donné à la figure 1.

Ces diverses fractions sont ensuite analysées par spectrométrie de masse afin d'identifier spécifiquement le contenu en acides aminés des peptides de chaque pic. Il a également été réalisé une analyse de séquençage, pour déterminer la séquence peptidique du peptide bioactif.

La détermination de la composition en acides aminés du principe actif selon l'invention a également été réalisée. Celle-ci est réalisée après hydrolyse acide et identification par chromatographie liquide haute pression à l'aide d'une pré-dérivation au PICT (phenylisothiocyanate).

Un exemple de composition en acides aminés de l'hydrolysat est donné dans le tableau suivant (en %) :

| Acides aminés | % |
|---|---|
| Alanine | 3,1 |
| Acide Aspartique | 4,7 |
| Arginine | 3,1 |
| Acide Glutamique | 32,8 |
| Glycine | 3,1 |
| Histidine | < 3,0 |

| | |
|---|---|
| Isoleucine | < 5,5 |
| Leucine | 6,2 |
| Lysine | < 2,2 |
| Phénylalanine | 4,5 |
| Proline | 10,9 |
| Serine | 4,7 |
| Thréonine | 3,1 |
| Tyrosine | < 3,6 |
| valine | 4,7 |
| Tryptophane | < 1,5 |

### Exemple 2 : Préparation d'un hydrolysat peptidique à partir de tubercules appartenant à l'espèce Solanum tuberosum

Les tubercules de pomme de terre (*Solanum tuberosum*) sont mis en solution dans 10 volumes d'eau en présence de 2 % de POLYCLAR® 10 (polyvinylpyrrolidone - PVPP - insoluble). Le mélange est ajusté à un pH compris entre 6 et 8 avec une solution aqueuse de soude 1 M. Une précipitation en milieu acide est ensuite réalisée. Le culot est remis en solution et après ajustement du pH, de la papaïne à 2 % est ajoutée dans le milieu réactionnel. L'hydrolyse est obtenue après 2 heures d'agitation à 55°C. On procède ensuite à l'inactivation de l'enzyme par chauffage de la solution à 80°C pendant 2 heures. Après centrifugation, la solution aqueuse surnageante correspondant à un hydrolysat brut de pomme de terre est récupérée. Les conditions d'hydrolyse ont été choisies de façon à permettre un enrichissement en peptide bioactif de 4 à 6 acides aminés contenant les résidus Gly, Leu et Acide glutamique.

Le procédé de purification de l'hydrolysat brut commence par des filtrations successives à l'aide de filtres-plaques Seitz-Orion de porosité décroissante (jusqu'à 0,2 µm) afin d'obtenir une solution jaune brillante et limpide, qualifié d'hydrolysat 1.

A cette étape, l'hydrolysat 1 de pomme de terre est caractérisé par un extrait sec titrant de 40 à 60 g/kg, un taux de protéines de 20 à 25 g/l et un taux de sucres de 1 à 3 g/l.

La nature protéique de l'hydrolysat 1 est mise en évidence après analyse par électrophorèse sur gel de polyacrylamide NuPAGE® Bis-Tris Pre-cast (Invitrogen). L'hydrolysat protéique de pomme de terre est chauffé à 70°C pendant 10 minutes dans des conditions réductrices dénaturantes dans un tampon de préparation d'échantillon NuPAGE® LDS. Une solution de NuPAGE® Antioxydant est ajoutée dans la cuve intérieure (cathode) pour éviter que les protéines réduites ne se ré-oxydent durant l'électrophorèse. La migration des protéines est réalisée dans du tampon de migration NuPAGE® MES avec le standard SeeBlue Plus2 comme marqueur de poids moléculaire. La coloration des protéines est effectuée à l'aide de Bleu de Coomassie® R-250. Dans ces conditions, on observe que les protéines obtenues ont un poids moléculaire inférieur à 6 kDa.

L'hydrolysat 1 est ensuite purifié afin de ne conserver que les peptides de poids moléculaire inférieurs à 5 kDa, à l'aide d'une filtration à flux tangentiel. Pour cela, l'hydrolysat 1 est pompé sous pression à travers un support Pellicon® équipé de cassette Pellicon® 2 Biomax 5 kDa. En fin de purification, on obtient un hydrolysat peptidique brillant et limpide. On procède ensuite à une phase de dilution afin d'obtenir un hydrolysat peptidique caractérisé par une teneur en protéines de 3,5 à 5,5 g/l. Cet hydrolysat peptidique correspond au principe actif selon l'invention.
Cet hydrolysat peptidiques est alors analysé en chromatographie liquide haute pression (HPLC) à l'aide d'un appareil HP1100 piloté par le logiciel ChemStation. La colonne utilisée lors de l'élution de l'hydrolysat est une Nucleosil® 300-5 C4 MPN (125 x 4 mn), permettant de chromatographier des protéines ayant des poids moléculaires de 0,2 à 25 kDa (selon des conditions identiques à l'exemple 1). Dans ces conditions chromatographiques, plusieurs fractions peptidiques ont pu être isolées.

Ces diverses fractions sont ensuite analysées par spectrométrie de masse afin d'identifier spécifiquement le contenu en acides aminés des peptides de chaque pic. Il a également été réalisé une analyse de séquençage, pour déterminer la séquence peptidique du peptide bioactif.

Un exemple de chromatogramme obtenu par CLHP (chromatographie en phase liquide à haute pression), avec mise en évidence du pic correspondant au peptide bioactif est donné à la figure 2.

La détermination de la composition en acides aminés du principe actif selon l'invention a également été réalisée. Celle-ci est réalisée après hydrolyse acide et identification par chromatographie liquide haute pression à l'aide d'une pré-dérivation au PICT (phenylisothiocyanate).

Un exemple de composition en acides aminés de l'hydrolysat est donné dans le tableau suivant (en %) :

| Acides aminés | % |
|---|---|
| Alanine | 7,8 |
| Acide Aspartique | 20,1 |
| Arginine | 7,3 |
| Acide Glutamique | 17,4 |
| Glycine | 7,3 |
| Histidine | 3,2 |
| Isoleucine | 9,1 |
| Leucine | 15,1 |
| Lysine | 11,4 |
| Phénylalanine | 8,7 |
| Proline | 7,7 |
| Serine | 8,7 |
| Thréonine | 9,1 |
| Tyrosine | 8,2 |
| valine | 10,5 |
| Tryptophane | 1,4 |

### Exemple 3 : Préparation d'un hydrolysat peptidique à partir de tourteaux de maïs (Zea mays L.)

Le tourteau de maïs (*Zea mays L*.) est mis en solution dans 10 volumes d'eau en présence de 2 % de POLYCLAR® 10 (polyvinylpyrrolidone - PVPP - insoluble). Le mélange est ajusté à un pH compris entre 6 et 8 avec une solution aqueuse de soude 1 M.

Après ajustement du pH, de la papaïne à 2 % est ajoutée dans le milieu réactionnel. L'hydrolyse est obtenue après 2 heures d'agitation à 55°C. On procède ensuite à l'inactivation de l'enzyme par chauffage de la solution à 80°C pendant 2 heures. Après centrifugation, la solution aqueuse surnageante correspondant à un hydrolysat brut de maïs est récupérée. Les conditions d'hydrolyse ont été choisies de façon à permettre un enrichissement en peptide bioactif de 4 à 6 acides aminés contenant les résidus Gly, Leu et Acide glutamique.

Le procédé de purification de l'hydrolysat brut commence par des filtrations successives à l'aide de filtres-plaques Seitz-Orion de porosité décroissante (jusqu'à 0.2 µm) afin d'obtenir une solution jaune, brillante et limpide, qualifié d'hydrolysat 1.

A cette étape, l'hydrolysat 1 de maïs est caractérisé par un extrait sec titrant de 20 à 30 g/kg, un taux de protéines de 20 à 25 g/l et un taux de sucres de 2 à 5 g/l.

La nature protéique de l'hydrolysat 1 est mise en évidence après analyse par électrophorèse sur gel de polyacrylamide NuPAGE® Bis-Tris Pre-cast (Invitrogen). L'hydrolysat protéique de maïs est chauffé à 70°C pendant 10 minutes dans des conditions réductrices, dénaturantes dans un tampon de préparation d'échantillon NuPAGE® LDS. Une solution de NuPAGE® Antioxydant est ajoutée dans la cuve intérieure (cathode) pour éviter que les protéines réduites ne se ré-oxydent durant l'électrophorèse. La migration des protéines est réalisée dans du tampon de migration NuPAGE® MES avec le standard SeeBlue Plus2 comme marqueur de poids moléculaire. La coloration des protéines est effectuée à l'aide de Bleu de Coomassie® R-250. Dans ces conditions, on observe des protéines de poids moléculaire inférieur à 6 kDa.

L'hydrolysat 1 est ensuite purifié en éliminant les protéines de haut poids moléculaire par ultrafiltration à l'aide de la cassette Pellicon® 2 Biomax 5 kDa afin de ne conserver que les composés de nature peptidique inférieurs à 5 kDa.

Après cette purification finale, on procède à une phase de dilution afin d'obtenir un hydrolysat peptidique caractérisé par un taux de protéines compris entre 3,5 et 5,5 g/l. Cet hydrolysat peptidique correspond au principe actif selon l'invention.

Cet hydrolysat peptidique est alors analysé en chromatographie liquide haute pression (CLHP) à l'aide d'un appareil HP1100 piloté par le logiciel ChemStation. La colonne utilisée lors de l'élution de l'hydrolysat est une Nucleosil® 300-5 C4 MPN (125 x 4 mn), permettant de chromatographier des protéines ayant des poids moléculaires de 0.2 à 25 kDa (selon un gradient de solvants approprié, identique à l'exemple 1). Dans ces conditions chromatographiques, plusieurs fractions peptidiques ont pu être isolées.

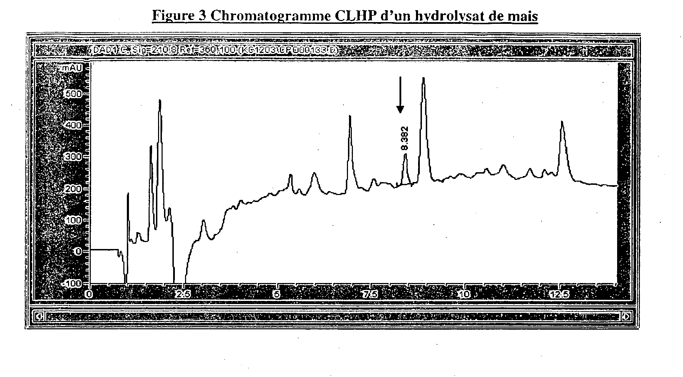

Un exemple de chromatogramme obtenu par CLHP (chromatographie en phase liquide à haute pression), avec mise en évidence du pic correspondant au peptide bioactif est donné à la figure 3

Ces diverses fractions sont ensuite analysées par spectrométrie de masse afin d'identifier spécifiquement le contenu en acides aminés des peptides de chaque pic. Il a également été réalisé une analyse de séquençage, pour déterminer la séquence peptidique du peptide bioactif.

La détermination de la composition en acides aminés du principe actif selon l'invention a également été réalisée. Celle-ci est réalisée après hydrolyse acide et identification par chromatographie liquide haute pression à l'aide d'une pré-dérivation au PICT (phenylisothiocyanate). Un exemple de composition en acides aminés de l'hydrolysat est donné dans le tableau suivant (%) :

| Acides aminés | % |
|---|---|
| Alanine | 9,4 |
| Acide Aspartique | 7,2 |
| Arginine | 3,6 |
| Acide Glutamique | 23,7 |
| Glycine | 3,6 |
| Histidine | 2,2 |
| Isoleucine | 4,5 |
| Leucine | 16,1 |
| Lysine | 2,2 |
| Phénylalanine | 6,7 |
| Proline | 10,3 |
| Serine | 6,3 |
| Thréonine | 4,0 |
| Tyrosine | 5,8 |
| valine | 5,4 |
| Tryptophane | < 0,5 |

### Exemple 4 : Préparation d'un hydrolysat peptidique à partir d'orge (Hordeum vulgare L.)

La farine d'orge (*Hordeum vulgare L*) est préparée de manière classique par broyage et tamisage du grain après séparation de son enveloppe. Elle est mise en solution dans 20 volumes d'eau en présence de 2 % de POLYCLAR® 10 (polyvinylpyrrolidone - PVPP - insoluble). Le mélange est ajusté à un pH compris entre 6 et 7,5 avec une solution aqueuse de soude 1 M.

Après ajustement du pH, il est rajouté 2% de bromélaïne dans le milieu réactionnel. L'hydrolyse est obtenue après 2 heures d'agitation à 50°C. On procède à l'inactivation de l'enzyme en chauffant la solution à 80°C pendant 2 heures. Après centrifugation, la solution aqueuse correspondant à l'extrait d'orge est récupérée.

Afin d'éliminer un maximum de sucre provenant de la dégradation de la cellulose, une autre voie d'extraction est également possible. L'orge est mis en solution dans 10 volumes d'eau et le pH de la solution est ajusté entre 4 et 5 avec une solution d'acide chlorhydrique 1 M. Après ajustement du pH, il est rajouté 2 % d'alpha-amylase dans le milieu réactionnel. L'hydrolyse est obtenue après 2 heures d'agitation à 50°C. En fin de réaction, le mélange réactionnel est filtré et on récupère le résidu d'extraction. Ce dernier est alors remis en solution dans 10 volumes d'eau en présence de 2 % de POLYCLAR® 10 (polyvinylpyrrolidone - PVPP - insoluble). Le pH est alors ajusté entre 6 et 7,5 avec une solution aqueuse de soude 1 M.

Après ajustement du pH, 2% de bromélaïne sont dans le milieu réactionnel. L'hydrolyse est obtenue après 2 heures d'agitation à 50°C. On procède à l'inactivation de l'enzyme en chauffant la solution à 80°C pendant 2 heures. Après centrifugation, la solution aqueuse correspondant à une première forme de l'hydrolysat d'orge est récupérée. Les conditions d'hydrolyse ont été choisies de façon à permettre un enrichissement en peptide bioactif de 3 à 5 acides aminés contenant les résidus Gly et Lys.

Le procédé de purification de l'hydrolysat brut commence par des filtrations successives à l'aide de filtres-plaques Seitz-Orion de porosité décroissante (jusqu'à 0,2 µm) afin d'obtenir une solution brillante et limpide. A cette étape, l'extrait d'orge est caractérisé par un poids sec de 10 à 13 g/kg, un taux de protéines de 5 à 7 g/l et un taux de sucres de 4 à 6 g/l.

La nature protéique de cet extrait est mise en évidence par électrophorèse sur gel de polyacrylamide. Pour cette analyse, il est utilisé les gels NuPAGE® Bis-Tris 4-12% (Invitrogen). L'hydrolysat peptidique d'orge est chauffé à 70°C pendant 10 minutes dans des conditions réductrices dénaturantes dans un tampon de préparation d'échantillon NuPAGE® LDS. Une solution de NuPAGE® Antioxydant est ajoutée dans la cuve intérieure (cathode) pour éviter que les protéines réduites se ré-oxydent durant l'électrophorèse. La migration des protéines est réalisée à l'aide du tampon de migration NuPAGE® MES avec les standards Mark 12 et Sharp standard (Invitrogen) comme marqueur de poids moléculaire. La coloration des protéines est effectuée à l'aide du kit de coloration à l'argent SilverXpress® (Invitrogen). Dans ces conditions, on observe des protéines ayant des poids moléculaires compris entre 85 kDa et 5 kDa ou inférieurs (limite de détection).

Cette forme de l'hydrolysat est alors purifiée en éliminant les protéines de haut poids moléculaire à l'aide de filtration à flux tangentiel.

Pour cela, la solution d'orge est pompée sous pression à travers un support Pellicon® équipée de cassette Pellicon® 2 Biomax 100 kDa. Ce 1er filtrat est récupéré pour être ensuite filtré à travers une seconde cassette Pellicon® 2 Biomax 50 kDa. Il est alors récupéré un second filtrat qui est encore élué à travers une cassette Pellicon® 2 Biomax 30 kDa. Le troisième filtrat est ensuite élué sur une cassette Pellicon® 2 Biomax 10 kDa. Celui-ci est enfin récupéré et re-concentré à l'aide d'une cassette Pellicon® 2 Biomax 3 kDa. En fin de purification, on obtient un hydrolysat peptidique d'orge de couleur beige, brillant et limpide. Il est caractérisé par un poids sec de 6 à 8 g/kg, une teneur en protéines de 5 à 7 g/l et une concentration en sucres inférieure à 1 g/l.

Après cette purification finale, on procède à une phase de dilution afin d'obtenir un hydrolysat peptidique caractérisé par un taux de protéines compris entre 3,5 et 5,5 g/l. Cet hydrolysat peptidique correspond au principe actif selon l'invention.

Cet hydrolysat peptidique est alors analysé en chromatographie liquide haute pression (CLHP) à l'aide d'un appareil HP1100 piloté par le logiciel ChemStation. La colonne utilisée lors de l'élution de l'hydrolysat est une Nucleosil® 300-5 C4 MPN (125 x 4 mn), permettant de chromatographier des protéines ayant des poids moléculaires de 0.2 à 25 kDa (dans des conditions identiques à l'exemple 1). Dans ces conditions chromatographiques, plusieurs fractions peptidiques ont pu être isolées.

La détermination de la composition en acides aminés du principe actif selon l'invention a également été réalisée. Celle-ci est réalisée après hydrolyse acide et identification par chromatographie liquide haute pression à l'aide d'une pré-dérivation au PICT (phenylisothiocyanate).

### Exemple 5 : Préparation d'un hydrolysat peptidique à partir de tourteau de colza (Brassica napus, var. oleifera)

Le tourteau de colza (*Brassica Napus*) est mis en solution dans 10 volumes d'eau en présence de 2 % de POLYCLAR® 10 (polyvinylpyrrolidone - PVPP - insoluble). Le mélange est ajusté à un pH compris entre 6 et 8 avec une solution aqueuse de soude 1 M.

Après ajustement du pH, de la bromélaïne à 2 % est ajoutée dans le milieu réactionnel. L'hydrolyse est obtenue après 2 heures d'agitation à 50°C. On procède ensuite à l'inactivation de l'enzyme par chauffage de la solution à 80°C pendant 2 heures. Après centrifugation, la solution aqueuse surnageante correspondant à un hydrolysat brut de colza est récupérée.. Les conditions d'hydrolyse ont été choisies de façon à permettre un enrichissement en peptide bioactif de 3 à 5 acides aminés contenant les résidus Gly et Lys.

Le procédé de purification de l'hydrolysat brut commence par des filtrations successives à l'aide de filtres-plaques Seitz-Orion de porosité décroissante (jusqu'à 0,2 µm) afin d'obtenir une solution brillante et limpide, qualifié d'hydrolysat 1.

A cette étape, l'hydrolysat 1 de colza est caractérisé par un extrait sec titrant de 30 à 40 g/kg, un taux de protéines de 25 à 35 g/l et un taux de sucres de 2 à 5 g/l.

La nature protéique de l'hydrolysat 1 est mise en évidence après analyse par électrophorèse sur gel de polyacrylamide NuPAGE® Bis-Tris Pre-cast (Invitrogen). L'hydrolysat protéique de colza est chauffé à 70°C pendant 10 minutes dans des conditions réductrices dénaturantes dans un tampon de préparation d'échantillon NuPAGE® LDS. Une solution de NuPAGE® Antioxydant est ajoutée dans la cuve intérieure (cathode) pour éviter que les protéines réduites ne se ré-oxydent durant l'électrophorèse. La migration des protéines est réalisée dans du tampon de migration NuPAGE® MES avec le standard SeeBlue Plus2 comme marqueur de poids moléculaire. La coloration des protéines est effectuée à l'aide de Bleu de Coomassie® R-250. Dans ces conditions, on observe 3 grandes familles de protéines : la 1ère famille correspond à des protéines de poids moléculaire de 65 à 35 kDa, la 2ème famille à des protéines de 25 à 15 kDa et la dernière famille à des protéines de poids moléculaire inférieur à 5kDa.

L'hydrolysat 1 est ensuite purifié en éliminant les protéines de haut poids moléculaire à l'aide de filtration à flux tangentiel. Pour cela, l'hydrolysat 1 est pompé sous pression à travers un support Pellicon® équipée de cassette Pellicon® 2 Biomax 50 kDa. Ce 1er filtrat est récupéré pour être ensuite filtré à travers une seconde cassette Pellicon® 2 Biomax 10 kDa. Il est alors récupéré un second filtrat qui est encore élué à travers une dernière cassette Pellicon® 2 Biomax 5 kDa. En fin de purification, on obtient un hydrolysat peptidique jaune-orangé, brillant et limpide. On procède à une phase de dilution afin d'obtenir un hydrolysat peptidique caractérisé par un extrait sec titrant de 6 à 8 g/kg, une teneur en protéines de 3,5 à 6 g/l et une concentration en sucres de 1 à 2 g/l. Cet hydrolysat peptidique correspond au principe actif selon l'invention.

Cet hydrolysat peptidique est alors analysé en chromatographie liquide haute pression (CLHP) à l'aide d'un appareil HP1100 piloté par le logiciel ChemStation. La colonne utilisée lors de l'élution de l'hydrolysat est une Nucleosil® 300-5 C4 MPN (125 x 4 mn), permettant de chromatographier des protéines ayant des poids moléculaires de 0.2 à 25 kDa (dans des conditions identiques à l'exemple 1). Dans ces conditions chromatographiques, plusieurs fractions peptidiques ont pu être isolées.

La détermination de la composition en acides aminés du principe actif selon l'invention a également été réalisée. Celle-ci est réalisée après hydrolyse acide et identification par chromatographie liquide haute pression à l'aide d'une pré-dérivation au PICT (phenylisothiocyanate).

### Exemple 6: Etude de l'effet protecteur de l'hydrolysat selon l'exemple 1 sur les cellules cutanées soumises à un stress oxydatif

Le but de cette étude est de déterminer l'effet protecteur de l'hydrolysat selon l'exemple 1 à 0,5 % vis-à-vis de kératinocytes humains normaux soumis à un stress oxydatif, provoqué par de l'eau oxygénée (H₂O₂) à 2 mM. Pour cela, des tests de viabilité cellulaire ont été réalisés par la technique au MTT.

Protocole : Les kératinocytes humains normaux sont traités avec de l'hydrolysat selon l'exemple 1 à 0,5 %, pendant 24 heures, soumis à un stress oxydatif provoqué par de l'H₂O₂ à 2 mM pendant 30 minutes, puis cultivés encore 24 heures en présence de la même concentration d'hydrolysat selon l'exemple 1 Des contrôles non traités par le peptide sont réalisés dans les mêmes conditions. A la fin de l'expérimentation, les cellules sont incubées dans une solution contenant 0,1 mg/ml de MTT (3-[4, 5-diméthylthiazol-2-yl]-2, 5-diphényltétrazolium, bromure). Ce composé est absorbé par les cellules vivantes puis métabolisé par les enzymes mitochondriales en un composé bleu violet, le formazan, qui sera dosé par spectrophotométrie à 540 nm. La densité optique (D.O.) est alors directement proportionnelle à l'activité enzymatique mitochondriale ainsi qu'au nombre de cellules vivantes.

Résultats : L'évaluation de la viabilité cellulaire par la technique du MTT montre que l'hydrolysat selon l'exemple 1 à 0,5 % augmente sensiblement la viabilité cellulaire des kératinocytes humains normaux.

Conclusion : L'hydrolysat selon l'exemple 1, protège efficacement les cellules cutanées contre les effets cytotoxiques d'un stress oxydatif.

### Exemple 7 : Etude de l'effet protecteur de l'hydrolysat selon l'exemple 2 sur les cellules cutanées agressées par un détergent

Le but de cette étude est de déterminer l'effet protecteur de l'hydrolysat selon l'exemple 2 vis-à-vis de kératinocytes humains normaux agressés par un détergent, en particulier le SDS. Pour cela, des tests de viabilité cellulaire ont été réalisés par la technique au MTT.

Protocole : Les kératinocytes humains normaux sont traités avec de l'hydrolysat selon l'exemple 2 à 0,5%, pendant 24 heures, soumis à un contact avec du SDS à 15 µg/ml pendant 24 heures, en présence de la même concentration d'hydrolysat selon l'exemple 2. Des contrôles non traités par le peptide sont réalisés dans les mêmes conditions. A la fin de l'expérimentation, les cellules sont incubées dans une solution contenant 0,1 mg/ml de MTT (3-[4, 5-diméthylthiazol-2-yl]-2, 5-diphényltétrazolium, bromure), selon le protocole décrit selon l'exemple 6.

Résultats : L'évaluation de la viabilité cellulaire par la technique du MTT montre que l'hydrolysat selon l'exemple 2 à 0,5 % augmente sensiblement la viabilité cellulaire des kératinocytes humains normaux.

Conclusion : L'hydrolysat selon l'exemple 2, protège efficacement les cellules cutanées contre l'agression par un détergent de type SDS.

### Exemple 8 : Etude ex vivo de l'effet de l'hydrolysat selon l'exemple 2 sur la sécrétion d'interleukine-I (IL-1 alpha) par des biopsies de peaux humaines

Le but de cette étude est de déterminer l'effet *ex vivo* de l'hydrolysat selon l'exemple 2 sur la production du médiateur cellulaire de l'inflammation IL-1 alpha, par des biopsies de peaux humaines cultivées en conditions standard ou soumises à un stress par des rayonnements UVB ou du SDS.

Protocole : Des biopsies de peau humaine de 5 mm de diamètre sont maintenues en culture à l'interface air-liquide, en présence de milieu de culture standard. Les échantillons sont traités avec de l'hydrolysat selon l'exemple 2 à 0,5%, pendant 24 heures, puis irradiés par des UVB (200 mJ/cm²) et remis en culture 24 heures, ou soumis à un contact avec du SDS à 2,5 % pendant 24 heures, en présence de la même concentration d'hydrolysat selon l'exemple 2. Des contrôles non traités par le peptide sont réalisés dans les mêmes conditions. A la fin de l'expérimentation, la quantité d'interleukine-1 alpha relarguée dans le milieu de culture est dosée par technique ELISA.

Résultats : La quantité d'IL-1 alpha relarguée par les biopsies de peau après un stress UVB ou SDS est diminuée, si les échantillons ont été traités par l'hydrolysat selon l'exemple 2

Conclusion : L'hydrolysat selon l'exemple 2, diminue sensiblement l'inflammation induite par les UBV ou le détergent SDS.

### Exemple 9 : Etude de l'effet protecteur de l'hydrolysat selon l'exemple 2 sur les cellules cutanées soumises à des rayonnements ultraviolet (UVB)

Le but de cette étude est de déterminer l'effet protecteur de l'hydrolysat selon l'exemple 2 vis-à-vis de kératinocytes humains normaux soumis à un stress par des rayonnements UVB. Pour cela, des tests de viabilité cellulaire ont été réalisés par la technique au MTT.

Protocole : Les kératinocytes humains normaux sont traités avec l'hydrolysat selon l'exemple 2 à 0,5 %, pendant 24 heures, irradiés par des UVB (50 mJ/cm²) puis cultivés encore 24 heures en présence de la même concentration d'hydrolysat selon l'exemple 2. Des contrôles non traités et irradiés sont réalisés dans les mêmes conditions. A la fin de l'expérimentation, les cellules sont incubées dans une solution contenant 0,1 mg/ml de MTT (3-[4, 5-diméthylthiazol-2-yl]-2, 5-diphényltétrazolium, bromure), et le MTT est dosé selon protocole décrit dans l'exemple 6.

Résultats : L'évaluation de la viabilité cellulaire par la technique du MTT montre que l'hydrolysat selon l'exemple 2 à 0,5 %, augmente sensiblement la viabilité cellulaire après irradiation par les UVB.

Conclusion : L'hydrolysat selon l'exemple 2, augmente la viabilité cellulaire et protège efficacement les cellules cutanées contre les effets cytotoxiques des rayonnements UVB.

### Exemple 10 : Etude de l'expression de la HMG-CoA réductase dans des biopsies de peau, en présence de l'hydrolysat selon l'exemple 1

Le but de cette étude est de déterminer l'influence l'hydrolysat selon l'exemple 1 à 1% sur l'expression de la HMG-CoA réductase.

Protocole : Des échantillons de peau humaine sont mis en culture à l'interface air/liquide. L'hydrolysat selon l'exemple 1 à 1 % est appliqué topiquement, puis les échantillons sont incubés durant 24 heures ou 48 heures.

Ces échantillons de peau sont ensuite fixés avec du formaldéhyde puis inclus dans la paraffine. Des coupes de 2 à 3 µm sont alors réalisées. L'immunomarquage est effectué après démasquage des sites spécifiques par traitement micro-onde puis incubation dans de la trypsine. L'immunomarquage est réalisé à l'aide d'un anticorps polyclonal de lapin spécifique de la HMG-CoA réductase (Millipore, Upstate), puis d'un anticorps secondaire, couplé à un marqueur fluorescent. Les coupes de peau sont alors examinées au microscope à Epi-fluorescence (Nikon Eclipse E600 microscope).

Résultats : Les observations microscopiques montrent une fluorescence plus forte dans les couches supérieures de l'épiderme des peaux traitées par l'hydrolysat selon l'exemple 1 à 1 %, par rapport au contrôle non traité.

Conclusion : L'hydrolysat selon l'exemple 1, stimule l'expression de la HMG-CoA réductase, dans les couches supérieures de l'épiderme.

### Exemple 11 : Etude de l'expression de la HMG-CoA réductase dans les kératinocytes humains normaux, en présence l'hydrolysat selon l'exemple 2

Le but de cette étude est de déterminer l'influence de l'hydrolysat selon l'exemple 2 sur l'expression de la HMG-CoA réductase dans les kératinocytes humains normaux.

Protocole : Des kératinocytes humains normaux en culture sont traités avec l'hydrolysat selon l'exemple 2 à 0,5 % pendant 24 ou 48 heures (le milieu contenant l'actif est changé toutes les 24 heures). Les cellules sont ensuite lavées, fixées au méthanol froid pendant 4 minutes à 4°C. Les cellules sont incubées en présence d'un anticorps polyclonal de lapin spécifique de la HMG-CoA réductase (Millipore, Upstate), puis d'un anticorps secondaire, couplé à un marqueur fluorescent. Les cellules sont alors examinées au microscope à Epi-fluorescence (Nikon Eclipse E600 microscope).

Résultats : Les observations microscopiques montrent une fluorescence cytoplasmique plus intense dans les cellules traitées par l'hydrolysat selon l'exemple 2 à 0,5 %.

Conclusion : L'hydrolysat selon l'exemple 2, stimule l'expression de la HMG-CoA réductase, dans les kératinocytes humains normaux.

### Exemple 12 : Préparation de compositions

### 1 - Crème protection solaire:

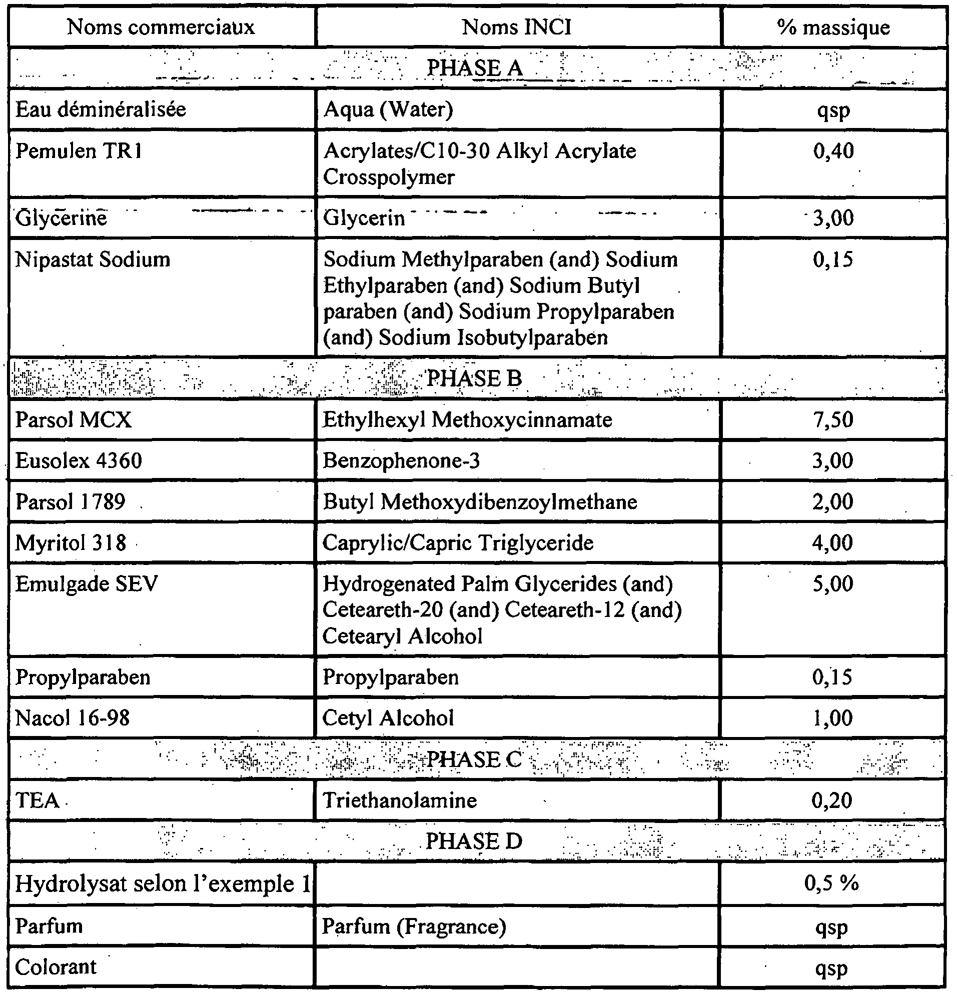

Les constituants de la phase A et de la phase B sont chauffés séparément entre 70°C et 75°C. La phase B est émulsionnée dans la phase A sous agitation. La phase C est ajoutée, à 45°C, en augmentant l'agitation. La phase D est ensuite additionnée lorsque la température se situe en dessous de 40°C. Lé refroidissement est poursuivi jusqu'à 25°C sous vive agitation.

### 2-Lait après-soleil :

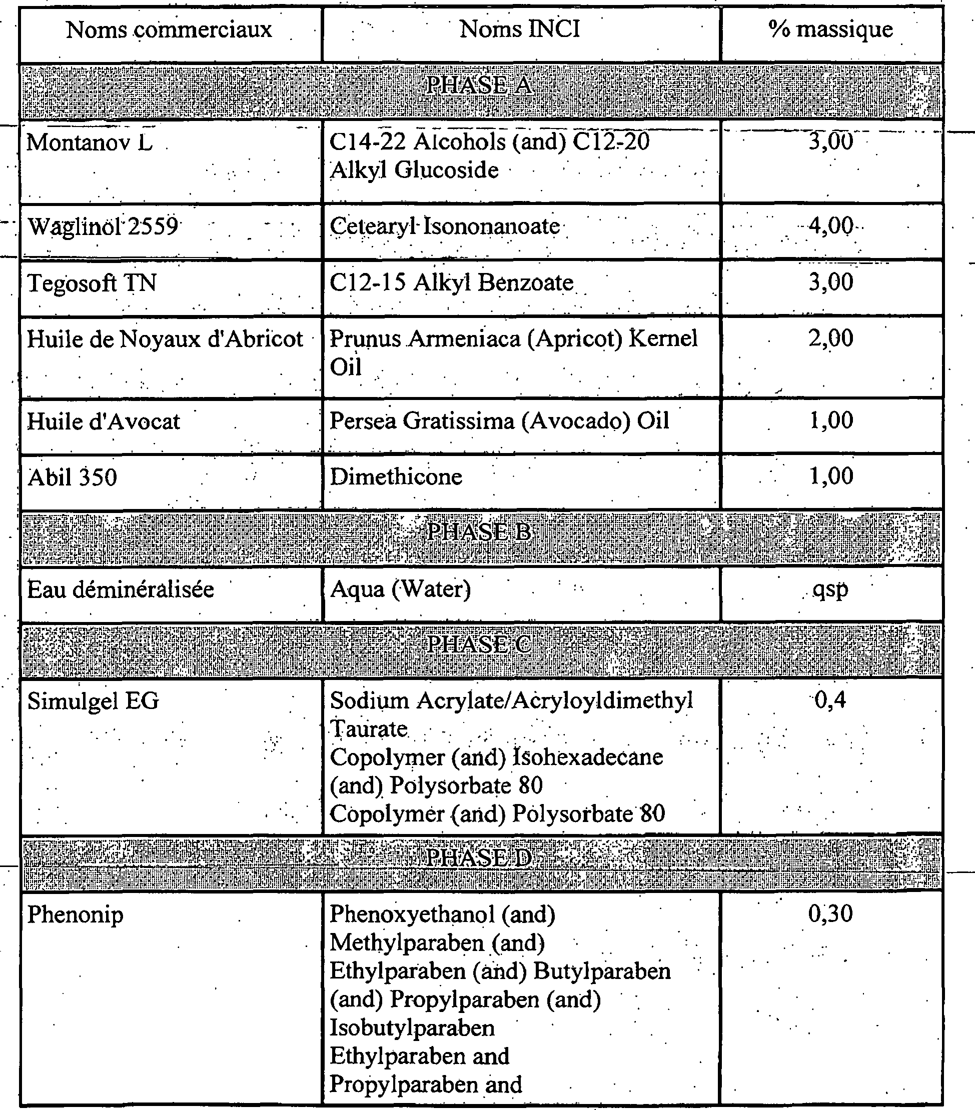

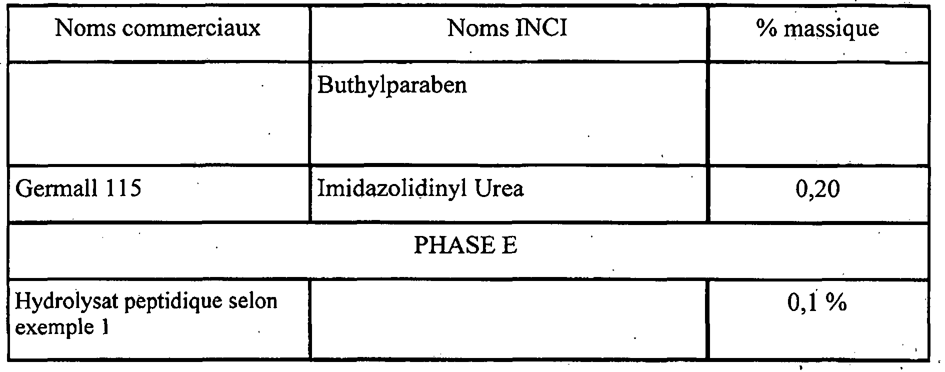

Préparer la phase A sous agitation. Incorporer la gomme xanthane progressivement, sous agitation défloculeuse. Les phases C et D seront incorporées une fois le gel terminé. La phase E, préparée préalablement jusqu'à parfaite dissolution de la DHA, sera rajoutée ensuite. Ajuster le pH si nécessaire à 4 - 4,5. Colorer et parfumer.

### 3 -Crème protectrice de jour :

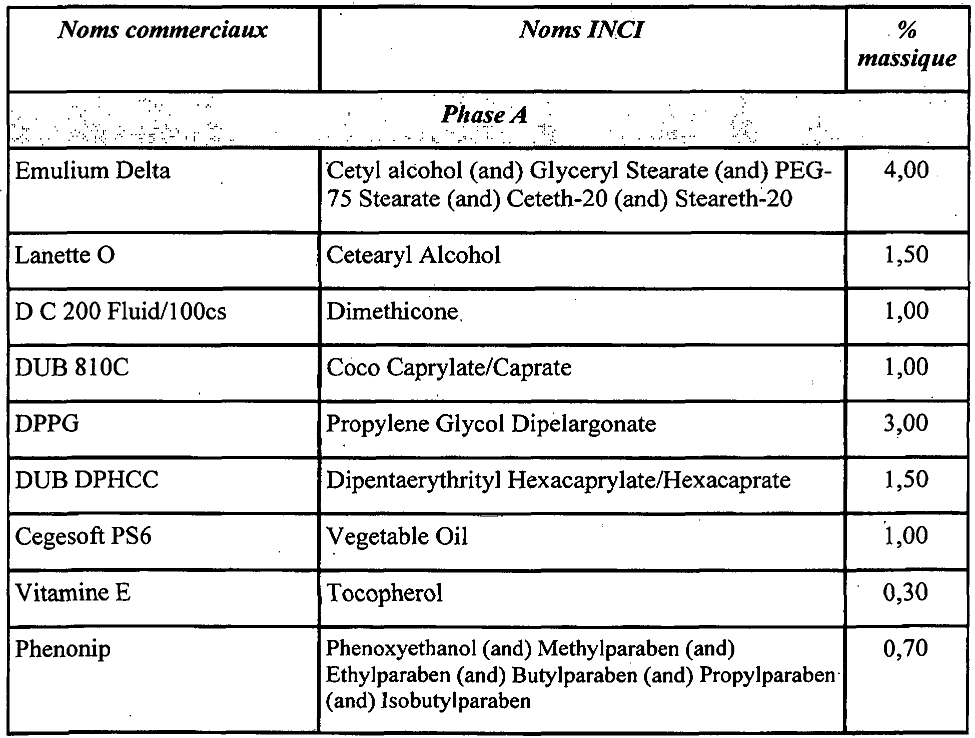

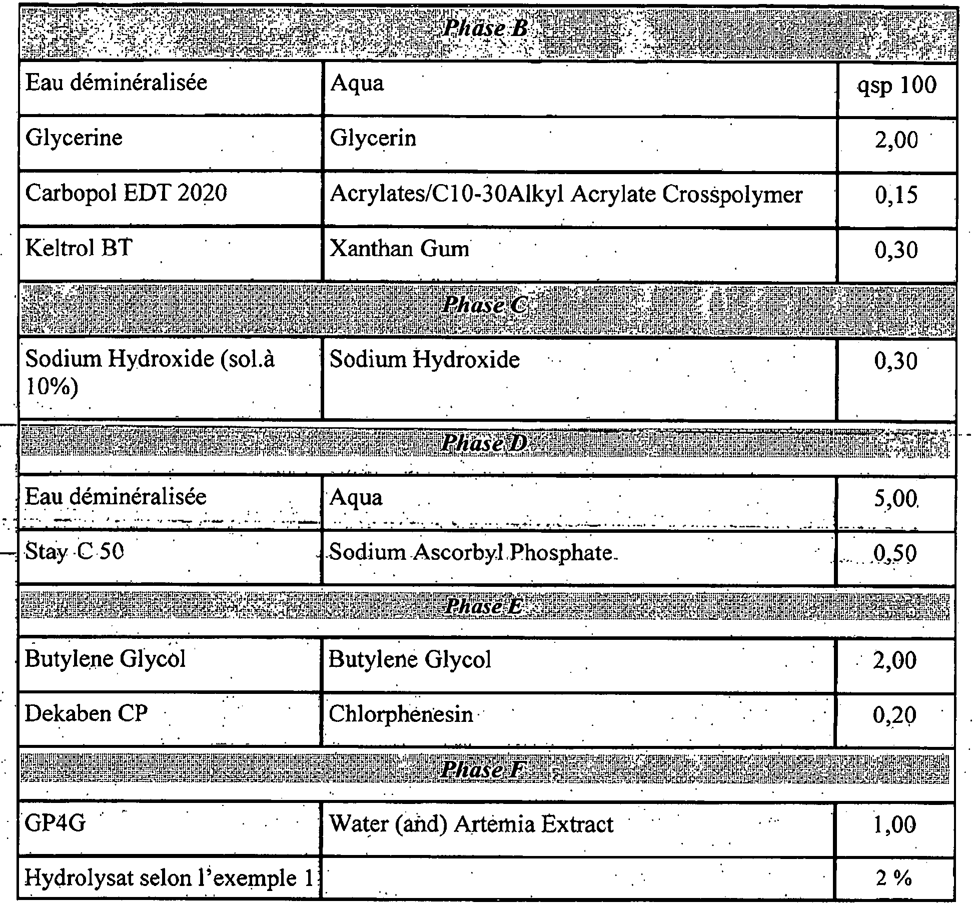

Préparer la phase A et chauffer à 75°C sous agitation. Préparer la phase B en dispersant le carbopol, puis la gomme xanthane sous agitation. Laisser reposer. Chauffer à 75°C.

A température, émulsionner A dans B sous agitation rotor-stator. Neutraliser avec la phase C sous agitation rapide. Après refroidissement à 40°C, additionner la phase D, puis la phase E. Le refroidissement est poursuivi sous agitation légère et la phase F rajoutée.

## Revendications

1. Composition comprenant une quantité efficace d'un hydrolysat peptidique enrichi en peptide bioactif capable de renforcer la fonction barrière de l'épiderme, ledit peptide bioactif contenant de 4 à 6 acides aminés dont au moins un résidu glycine, un résidu leucine et un résidu acide glutamique, en tant que principe actif activateur de la HMG-CoA réductase, pour son utilisation pour apaiser les peaux sensibles, pour protéger la peau contre les agressions extérieures, ou pour prévenir ou lutter contre les irritations cutanées.

2. Composition selon la revendication 1, **caractérisée en ce que** les agressions extérieures sont les rayonnements UV ou les oxydants.

3. Composition selon l'une des revendications 1 ou 2, **caractérisée en ce que** l'hydrolysat peptidique provient de l'hydrolyse de plantes choisies parmi l'épeautre (*Triticum monococum*), la pomme de terre (*Solanum tuberosu*m), le maïs *(Zea mayz L.*), l'orge (*Hordeum vulgare L*.) ou le colza (*Brassica napus*).

4. Composition selon l'une des revendications précédentes, **caractérisée en ce que** le peptide bioactif est de formule générale (I) :
X₁-[Gly, Glu, Leu]- X₂-X₃
dans laquelle,
X₁ est l'alanine, la valine, l'isoleucine ou aucun acide aminé,
X₂ est la serine ou la thréonine,
X₃ est la leucine, l'isoleucine ou aucun acide aminé.

5. Composition selon la revendication 4, **caractérisée en ce que** le peptide bioactif est de séquence :
(SEQ ID n°1) Ala-Glu-Gly-Leu-Ser-Ile
(SEQ ID n°2) Leu-Gly-Glu-Ser-Leu
(SEQ ID n°3) Val-Gly-Glu-Leu-Thr
(SEQ ID n°4) Ile-Gly-Glu-Leu-Ser
(SEQ ID n°5) Ala-Gly-Glu-Leu-Ser
(SEQ ID n°6) Gly-Glu-Leu-Thr-Ile
(SEQ ID n°7) Gly-Glu-Leu-Ser.

6. Composition selon l'une des revendications précédentes, **caractérisée en ce que** l'hydrolysat peptidique contient entre 0,5 et 5,5 g/l de composés de nature peptidique.

7. Composition selon l'une des revendications précédentes, **caractérisée en ce que** l'hydrolysat peptidique est présent en une quantité représentant de 0,0001 % à 20 % du poids total de la composition.

8. Composition selon la revendication 7, **caractérisée en ce que** l'hydrolysat peptidique est présent en une quantité représentant de 0,05 % à 5 % du poids total de la composition.

9. Composition selon l'une des revendications précédentes, **caractérisée en ce qu'**elle se présente sous une forme adaptée à l'application par voie topique.

10. Composition selon l'une des revendications précédentes, **caractérisée en ce qu'**elle comprend au moins un autre principe actif à effet secondaire irritant favorisant l'action de l'hydrolysat peptidique choisi parmi l'acide glycolique, l'acide lactique, l'acide malique, l'acide citrique, l'acide mandélique, l'acide salicylique, l'acide ascorbique, la vitamine C, le rétinol, le rétinal, l'acide rétinoïque, le minoxidil, les sels de lithium, le para-phénylène-diamine (p-PDA), le N-phényl p-PDA, le toluène 2,5-diamine sulfate, la méta-phénylène diamine, le toluène 3,4-diamine, l'ortho-phénylène diamine, l'hydroquinone, l'hydrolysat peptidique étant destiné à prévenir ou lutter contre les irritations cutanées provoquées par le principe actif irritant.

11. Composition pharmaceutique comprenant un hydrolysat peptidique enrichi en peptide bioactif capable de renforcer la fonction barrière de l'épiderme, ledit peptide bioactif contenant de 4 à 6 acides aminés dont au moins un résidu glycine, un résidu leucine et un résidu acide glutamique, pour son utilisation pour prévenir ou traiter les inflammations cutanées telles que les érythèmes, en particulier les érythèmes dus aux ultraviolets (UV), ou le prurit, l'urticaire, les piqûres d'insectes, les allergies, ou encore l'alopécie dans ses phases inflammatoires.

## Patentansprüche

1. Zusammensetzung, umfassend eine wirksame Menge eines peptidischen Hydrolysats, angereichert mit bioaktivem Peptid, das dazu in der Lage ist, die Barrierefunktion der Epidermis zu verstärken, wobei das bioaktive Peptid von 4 bis 6 Aminosäuren enthält, darunter mindestens einen Glycinrest, einen Leucinrest und einen Glutaminsäurerest als Wirkstoff, der die HMG-CoA-Reduktase aktiviert, für die Verwendung zur Linderung von sensibler Haut, zum Schutz der Haut vor äußeren Aggressionen oder zur Verhinderung oder Bekämpfung von Hautreizungen.

2. Zusammensetzung nach Anspruch 1, **dadurch gekennzeichnet, dass** die äußeren Aggressionen UV-Strahlen oder Oxidationsmittel sind.

3. Zusammensetzung nach einem der Ansprüche 1 oder 2, **dadurch gekennzeichnet, dass** das peptidische Hydrolysat aus der Hydrolyse von Pflanzen stammt, ausgewählt aus dem Dinkel (*Triticum monococum*), der Kartoffel (*Solanum tuberosum*), dem Mais (*Zea mayz L.*), der Gerste (*Hordeum vulgare L.*) oder dem Raps (*Brassica napus*).

4. Zusammensetzung nach einem der vorhergehenden Ansprüche, **dadurch gekennzeichnet, dass** das bioaktive Peptid die folgende allgemeine Formel (I) aufweist:
X₁-[Gly, Glu, Leu]-X₂-X₃
wobei:
X₁ Alanin, Valin, Isoleucin oder keine Aminosäure ist,
X₂ Serin oder Threonin ist,
X₃ Leucin, Isoleucin oder keine Aminosäure ist.

5. Zusammensetzung nach Anspruch 4, **dadurch gekennzeichnet, dass** das bioaktive Peptid die Folgende Sequenz aufweist:
(SEQ ID Nr. 1) Ala-Glu-Gly-Leu-Ser-Ile
(SEQ ID Nr. 2) Leu-Gly-Glu-Ser-Leu
(SEQ ID Nr. 3) Val-Gly-Glu-Leu-Thr
(SEQ ID Nr. 4) IIe-Gly-Glu-Leu-Ser
(SEQ ID Nr. 5) Ala-Gly-Glu-Leu-Ser
(SEQ ID Nr. 6) Gly-Glu-Leu-Thr-Ile
(SEQ ID Nr. 7) Gly-Glu-Leu-Ser.

6. Zusammensetzung nach einem der vorhergehenden Ansprüche, **dadurch gekennzeichnet, dass** das peptidische Hydrolysat zwischen 0,5 und 5,5 g/l Verbindungen peptidischer Art umfasst.

7. Zusammensetzung nach einem der vorhergehenden Ansprüche, **dadurch gekennzeichnet, dass** das peptidische Hydrolysat in einer Menge vorhanden ist, die 0,0001 % bis 20 % des Gesamtgewichts der Zusammensetzung darstellt.

8. Zusammensetzung nach Anspruch 7, **dadurch gekennzeichnet, dass** peptidische Hydrolysat in einer Menge vorhanden ist, die 0,05 % bis 5 % des Gesamtgewichts der Zusammensetzung darstellt.

9. Zusammensetzung nach einem der vorhergehenden Ansprüche, **dadurch gekennzeichnet, dass** sie eine Form aufweist, die für die Anwendung auf topischem Weg ausgelegt ist.

10. Zusammensetzung nach einem der vorhergehenden Ansprüche, **dadurch gekennzeichnet, dass** sie mindestens einen weiteren Wirkstoff mit reizender Nebenwirkung umfasst, der die Wirkung des peptidischen Hydrolysats fördert, ausgewählt aus der Glykolsäure, der Milchsäure, der Apfelsäure, der Zitronensäure, der Mandelsäure, der Salicylsäure, der Ascorbinsäure, dem Vitamin C, dem Retinol, dem Retinal, der Retinsäure, dem Minoxidil, den Lithiumsalzen, dem Paraphenylendiamin (p-PDA), dem N-Phenyl-p-PDA, dem Toluen-2,5-Diamindsulfat, dem meta-Phenylendiamin, dem Toluen-3,4-Diamin, dem Orthophenylendiamin, dem Hydrochinon, wobei das peptidische Hydrolysat ausgelegt ist, um Hautirritationen, hervorgerufen vom reizenden Wirkstoff, vorzubeugen oder diese zu bekämpfen.

11. Zusammensetzung, umfassend ein peptidisches Hydrolysat, angereichert mit bioaktivem Peptid, das dazu in der Lage ist, die Barrierefunktion der Epidermis zu verstärken, wobei das bioaktive Peptid von 4 bis 6 Aminosäuren enthält, darunter mindestens einen Glycinrest, einen Leucinrest und einen Glutaminsäurerest, für die Verwendung zur Vorbeugung oder Behandlung von Hautentzündungen wie z.B. den Erythemen, insbesondere den Erythemen aufgrund der Ultraviolettstrahlen (UV), oder dem Pruritus, der Urtikaria, den Insektenstichen, den Allergien oder auch der Alopezie in seinen entzündlichen Phasen.

## Claims

1. Composition comprising an effective quantity of a peptidic hydrolysate enriched with bioactive peptide capable of reinforcing the barrier function of the epidermis, said bioactive peptide containing 4 to 6 amino acids including at least a glycine residue, a leucine residue and a glutamic acid residue, as an active substance activating HMG-CoA reductase, for use for relieving sensitive skins, for protecting the skin against external attacks, or for preventing or combating skin irritations.

2. Composition according to claim 1, **characterised in that** the external attacks are UV rays or oxidants.

3. Composition according to any of claims 1 or 2, **characterised in that** the peptidic hydrolysate is obtained from the hydrolysis of plants chosen from Einkorn wheat (*Triticum monococcum*), potato (*Solanum* tuberosum), corn (*Zea mayz L.*), barley (*Hordeum vulgare L*.) or rapeseed *(Brassica napus*).

4. Composition according to any of the above claims, **characterised in that** the bioactive peptide has the general formula (I):
X₁-[Gly, Glu, Leu]-X₂-X₃
wherein,
X₁ is alanine, valine, isoleucine or no amino acid,
X₂ is serine or threonine,
X₃ is leucine, isoleucine or no amino acid.

5. Composition according to claim 4, **characterised in that** the bioactive peptide has the sequence:
(SEQ ID No. 1) Ala-Glu-Gly-Leu-Ser-Ile
(SEQ ID No. 2) Leu-Gly-Gla-Ser-Leu
(SEQ ID No. 3) Val-Gly-Glu-Leu-Thr
(SEQ ID No. 4) Ile-Gly-Glu-Leu-Ser
(SEQ ID No. 5) Ala-Gly-Glu-Leu-Ser
(SEQ ID No. 6) Gly-Glu-Leu-Thr-Ile
(SEQ ID No. 7) Gly-Glu-Leu-Ser.

6. Composition according to any of the above claims, **characterised in that** the peptidic hydrolysate contains between 0.5 and 5.5 g/l of peptidic compounds.

7. Composition according to any of the above claims, **characterised in that** the peptidic hydrolysate is present in a quantity representing from 0.0001% to 20% of the total weight of the composition.

8. Composition according to claim 7, **characterised in that** the peptidic hydrolysate is present in a quantity representing from 0.05% to 5% of the total weight of the composition.

9. Composition according to any of the above claims, **characterised in that** it is presented in a form suitable for topical application.

10. Composition according to any of the above claims, **characterised in that** it comprises at least one further active substance having an irritant side effect promoting the action of the peptidic hydrolysate chosen from glycolic acid, lactic acid, malic acid, citric acid, mandelic acid, salicylic acid, ascorbic acid, vitamin C, retinol, retinal, retinoic acid, minoxidil, lithium salts, para-phenylene-diamine (p-PDA), N-phenyl p-PDA, toluene 2,5-diamine sulphate, meta-phenylene diamine, toluene 3,4-diamine, ortho-phenylene diamine, hydroquinone, the peptidic hydrolysate being intended to prevent or combat skin irritations caused by the irritant active substance.

11. Pharmaceutical composition comprising a peptidic hydrolysate enriched with bioactive peptide capable of reinforcing the barrier function of the epidermis, said bioactive peptide containing 4 to 6 amino acids including at least a glycine residue, a leucine residue and a glutamic acid residue, for use for preventing or treating skin inflammations such as erythema, particularly erythema caused by ultraviolet (UV) rays, or pruritus, urticaria, insect bites, allergies, or alopecia in the inflammatory phases thereof.
